# EUROPEAN PATENT SPECIFICATION

(11) **EP 1 239 785 B1**
(45) Date of publication and mention of the grant of the patent: **01.09.2004**
(21) Application number: 99957834.7
(22) Date of filing: 20.12.1999
(51) Int. Cl.: A61B 17/70

(54) **DEVICE FOR THE STABILISATION OF TWO ADJACENT VERTERBRAL BODIES OF THE SPINE**
ANORDNUNG ZUR STABILISIERUNG ZWEIER NEBENEINANDERLIEGENDEN WIRBELN DER WIRBELSAÜLE
DISPOSITIF POUR LA STABILISATION DE DEUX CORPS VERTEBRAUX ADJACENTS DE LA COLONNE VERTEBRALE

(43) Date of publication of application: 18.09.2002
(73) Proprietor: SYNTHES AG Chur, 7002 Chur (CH)
(72) Inventor: MULHOLLAND, Robert C., Sherwood, Nottingham NG5 4DD (GB); SENGUPTA, Dilip K., Nottingham NG7 2UH (GB)
(74) Representative: Lusuardi, Werther Giovanni, Dr.
(86) International application number: PCT/CH1999/000612
(87) International publication number: WO 2001/045576

(56) References cited:
- EP-A- 0 669 109
- DE-A- 3 807 335
- DE-U- 9 112 466
- FR-A- 2 715 057

## Description

This invention concerns a device in accordance with the pre-characterising portion of Claim 1, which is known for example from document EP-A-0 669 109.

The human spine consists of blocks of bony vertebra with intervening blocks of soft-tissue, the intervertebral discs. These functional elements allow the spine to be flexible, and to transfer load evenly over the lower vertebra, despite variation in the position of the upper vertebra, They function as a fluid filled bag between each vertebra. However, with disc degeneration, they become anisotropic and transmit load irregularly over the endplate and this irregular loading pattern in different positions of the spine is associated with back pain.

Conventional treatment of this disorder is to fuse the two vertebrae together. This method of treatment is not attended by a high level of success. This is in part related to the stiff segment created by the fusion operation, which produces problems to the adjacent spinal segments above and below. The ideal solution would be one that allows normal load transmission and also allows movement as achieved with artificial joints elsewhere in the human body. However, the artificial disc prostheses, which have been proposed so far, have not to date produced any better results than fusion. This is in part because insertion of an artificial disc involves major surgery, and there is the difficulty in accurately placing the device to ensure that the segmental movement of the facet joints remains normal.

There are two further devices described in the treatemnt of low back pain, known as "soft-stabilisation" in which pedicle screws are placed in the vertebrae, and a stretchable ligament is attached behind, at their head portions. In both the devices, the restraining ligaments are well behind the axis of flexion/extension. In one of these systems (Dyesys-Sulzer), whilst excessive lordosis is prevented by a cylinder embracing the ligament, the actual distraction of the posterior disc space can only be achieved by producing flexion of the segment. It is known that a kyphotic segment in the lumbar spine can produce back pain.

In the other of these systems (Graf ligament), distraction of the anterior disc space and creation of physiological lordosis is accompanied by compression of the posterior part of the disc. This results in narrowing of the diameter of the exit foramen of the nerve root, and also increases load transfer to the posterior part of the disc. It is known that increased load transfer by the posterior annulus can produce annular tear. Neither system addresses therefore the important aims of producing uniform disc distraction and the creating a normal loading pattern across the end-plate of the vertebrae.

The invention as claimed, aims at solving the problems described above.

The present invention provides a device as defined in Claim 1. By introduction of a fulcrum, lying close to the axis of flexion and extension of the spinal motion segment, the posteriorly placed ligament distracts the whole disc, and the fulcrum itself becomes a load-bearing structure. This reduces the load over the disc, and makes it an even distribution of load across the end-plate throughout the range of movement of the motion segment, that is allowed by the implant.

Clinically the ligament is applied to the assembly under tension and the fulcrum under compression at the resting position of the spine. The screws are introduced posteriorly through the pedicles on either side, so that there is an assembly of screws, fulcrums and ligaments on right and left side in the posterior aspect of the vertebrae to be stabilised. the same system can also be extended over more than one motion segment, if necessary.

The fulcrum lies closer to the axis of movement of flexion-extension of the lumbar spine. When the assembly is introduced into the spine, it allows movement of the spine in flexion-extension, as well as in other directions. During flexion-extension, the range of movement of the fulcrum is less than that of the ligament, since the former lies closer to the axis of the movement.

The ligament actually elongates during flexion and recoils during extension. During flexion, the fulcrum bends, and also to some extent, compresses itself. These movements are reversed in extension. The two ends of the fulcrum, being tightly fixed to the pedicle screws, prevent any movement between the pedicle screws and the fulcrum at their junction.

The pedicle screws will preferably be made up of titanium and with diameters of 5, 6, 7, and 8 mm. The pedicle screw has a head portion and a threaded shaft portion. The length of the threaded shaft portion of the screw is preferably between 35 and 55 mm, with 5 mm increments. The length of the head portion is preferably of different sizes, e.g. 20, 30 and 40mm.

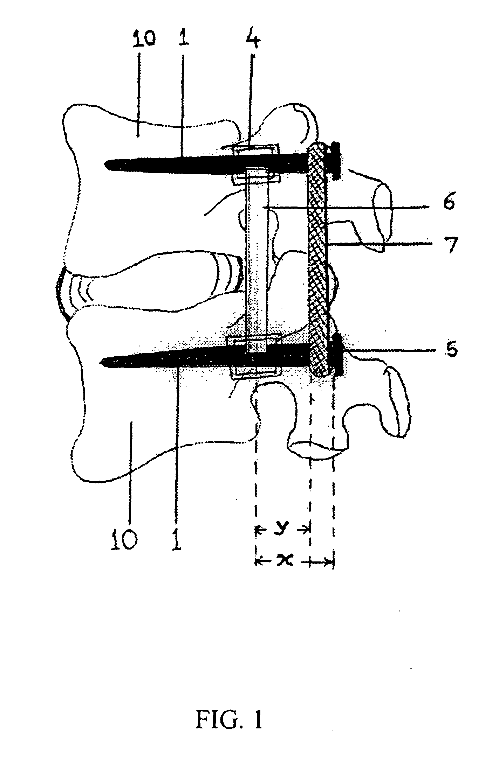

The distance x is preferably in the range of 10 - 20 mm and the distance y is preferably in the range of 7 to 17 mm (Fig. 1).

The fulcrum should (at least partially) be compressible in the longitudinal direction. In a preferred embodiment the fulcrum consists of a longitudinal main body of which at least its middle portion consists of a flexible material and two end portions consisting of a rigid material (e.g. the same as that of the pedicle screws). The two end portions can be designed as a sleeve over the main body.
The main body of said fulcrum is preferably made of a flexible polymer, e.g. a high-density polyethylene (HDP) with a modulus of elasticity between 17'000 to 28'000 kg/cm² or a poly tetra-fluoro ethylene (PTFE) with modulus of elasticity between 3000 to 6500 kg/cm². The outer diameter at the metallic ends of the fulcrum is preferably between 5, 6, or 7 mm. The flexibility of the fulcrum will depend on the design and the diameter as well.

The fulcrum is preferably covered with a fabric sleeve. This has the advantage that it can resist the wear resulting from friction of the relatively soft material of the fulcrum against the bone and the soft tissues around, in the spine.
Preferably the fulcrum is drum-shaped in order to prevent buckling during bending.

Preferably the head portions of the pedicle screws are provided with a fixation mechanism for accommodating and fixing an end portion of the fulcrum.
The head portion of the pedicle screw is provided with a smooth seat for receiving the ligament , a groove for receiving the end portion of the fulcrum, and a threaded portion for receiving a locking nut.
In a preferred embodiment the assembly has a fastening mechanism to tighten the end portion of the fulcrum to the head of the pedicle screws. The fastening mechanism preferably comprises of a sleeve which can be passed over the end portion of the fulcrum, after the latter is seated into the groove in the head portion of the pedicle screw

In a further preferred embodiment the assembly comprises of a locking nut which can be tightened over the corresponding threaded part in the head of the pedicle screw , fastening the sleeve and the end portion of the fulcrum to the head of the pedicle screw.

In a further preferred embodiment the ligament has the shape of a closed loop, which can be passed over the second end of the pedicle screw, to sit on the smooth part of the head portion of the pedicle screw. It may further comprise a cap nut which can be screwed into the second end of the pedicle screw to retain the ligament in place.

The ligament is preferably made up with braided polyester. The diameter of the cross section of the ligament can vary between 3 and 7 mm. The breaking strength should be between 2000 N and 5000 N load, depending on the diameter of the ligament, and rate of loading. The strain will be between 10 and 30 % under physiological loading.

**In the drawings:**
- Fig. 1: Shows a schematic representation of a longitudinal section through the assembly according to a preferred embodiment of the invention;
- Fig. 2a: shows a side view of the fulcrum, attached to a pedicle screw in the assembly according to a preferred embodiment of the invention;
- Fig. 2b: shows the pedicle screw, the fastening mechanism of the fulcrum to it, and the cap nut, before they are assembled together;
- Fig. 2c: shows the profile of the pedicle screw and the sleeve of the festening mechanism;
- Fig. 3: shows the assembly of the pedicle screw, the fulcrum, and the ligament together for one side of the spinal segment; and
- Fig. 4: shows the assembly applied to one spinal motion segment, shown in a plastic model of the lumbar spine, from a posterior oblique view.

The assembly as shown in Fig. 1 is used for the stabilisation of two adjacent vertebral bodies 10 of the spine. It comprises two pedicle screws 1, having a threaded shaft 2 with a tapering first end 3 for introduction into the vertebral bodies 10 and a head portion 4 with a second end 5 which are shown in more detail in Figs. 2b and 2c.

The pedicle screw is made up of titanium and its diameter is 7 mm (which may vary between 5 and 8 mm). The length of the threaded portion of the pedicle screw is 45 mm (which may vary between 35 and 55 mm, with 5 mm increments). The length of the head portion up to its said second end is 30 mm (which may vary between 20 and 40 mm).

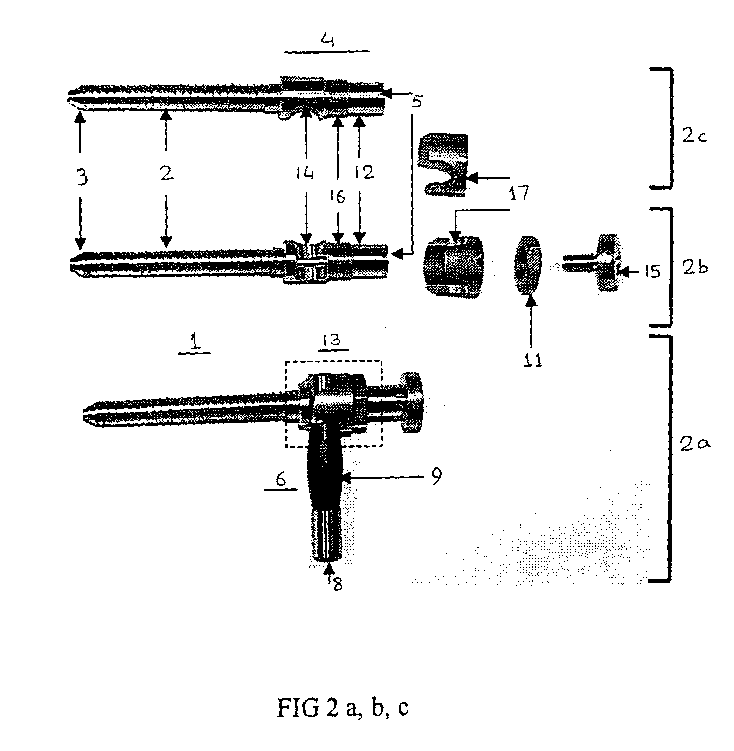

As shown in Fig. 2a, a flexible longitudinal fulcrum 6 with two end portions 8, can be disposed transversely to the pedicle screws 1, and fixed with its end portions 8 to the head portion 4 of the two pedicle screws 1, at a distance of 15 mm (which may vary between 10 and 20 mm) from the said second end 5 as shown in Fig. 1. The main body 9 of the fulcrum 6 consists of a flexible material, similar to 'high density polyethylene' (HDP) with a modulus of elasticity of 22'500 kg/cm² (which may vary between 17'000 and 28'000 kg/cm² or 'poly tetra-fluoro ethylene (PTFE) with a modulus of elasticity of 4 '750 kg/cm² (which may vary between 3'000 amd 4'500 kg/cm²). The two end portions 8 consist of a metallic sleeve, between 15 and 20 mm long, over the flexible material of the fulcrum. The outer diameter of these metallic end portions 8 are 6 mm (which may vary between 5 and 8 mm). The fulcrum 6 is drum shaped, with larger diameter in its middle compared to the ends. The fulcrum is at least partially compressible in the longitudinal direction.

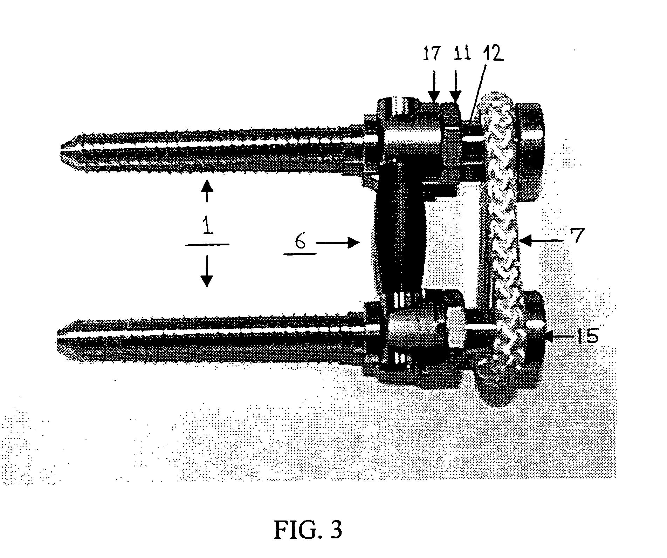

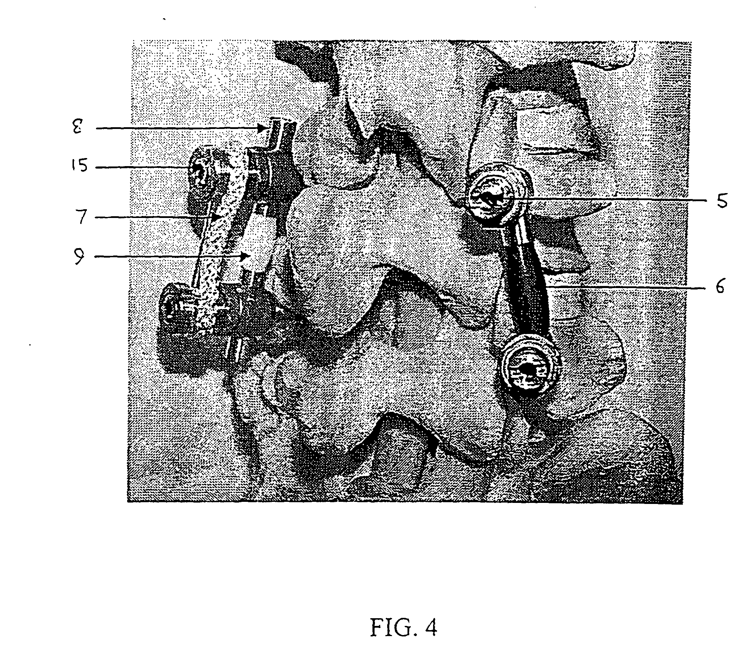

As shown in Figs. 3 and 4, the ligament 7 has teh shape of a loop, which can be passed over the second end 5 of the pedicle screw, to sit on the smooth part 12 of the head portion 4 of the pedicle screw. The diameter of the cross section of the ligament is 5 mm (which may vary between 3 and 7 mm) and its breaking strength is 3'500 N load in tension (which may vary between 2'000 and 4'500 N). The strain of the ligament is 20 % under physiological loading.

The elastic ligament 7 made up of braided polyester. The ligament 7 is disposed transversely to the said pedicle screws 1, and fixed to the head portions 4 of the pedicle screws 1 at a distance of 12 mm (whihc may vary between 7 and 17 mm) from the groove 14 for the fulcrum in the nead of the pedicle screw.

Alternatively the ligament may consist of a single length flexible cord, which is made up with an outer layer of braided polyester, and an inner core of stretchable material like Dyneema (which is registered trademark of DSM High Performance Fibres B.V., Netherlands) or similar. The single length version (as opposed to the above described loop version) of the ligament has metal sleeves at its ends for fastening it to the second end (5) of the pedicle screws.

As shown in Fig. 2c the head portions 4 of the pedicle screws 1 are provided with a fixation mechanism 12,14,16 for accommodating and fixing an end portion 8 of the fulcrum 6. The head portion 4 of the pedicle screw 1 is provided with a smooth seat 12 at its free and for receiving the ligament 7 (Fig. 1), a groove 14 for receiving an end portion 8 of the fulcrum 6, and a threaded portion 16 locate between the groove 14 and the smooth seat 12 for receiving a locking nut 11.

As shown in Fig. 2 c the head portion 4 of the pedicle screws 1 consists of three segments 12,14,16 for accommodation and fixation of the end portions 8 of the fulcrum 6. These are , a smooth seat 12 at its free and for receiving teh ligament 7, a groove 14 for receiving the end portion 8 of the fulcrum 6, and a threaded portion 16, located between the smooth seat and the groove, for receiving the locking nut 11.

As shown in Fig, 2a the end portion 8 of the fulcrum 6 is fastened to the head portion 4 of the pedicle screw with a sleeve 17 and a nut 11. The fastening mechanism 13 is shown in more detail in Fig. 2b. It comprises a sleeve 17 which can be passed over the end portions 8 of the fulcrum 6 after the latter is seated into the grooved 14 in the head portion 4 of the pedicle screw. It further comprises a locking nut 11, which can be tightened over the corresponding threaded portion 16 in the head portion 4 of the pedicle screw, fastening the sleeve 17 and teh end portion 8 of the fulcrum, to the pedicle screw. Finally a cap nut 15 can be screwed into the second end 5 of the pedicle screw, to retain the ligament in place.

In Fig. 3 the completed assembly is represented with the fulcrum 6 fixed to the two pedicle screws 1 and the ligament 7 wound and secured on the head portions 4 of the pedicle screws 1.

In Fig. 4 two pairs of assemblies are shown implanted in the spine model. On the right hand assembly only the fulcrum 6 has been fixed to the head portion 4 of the pedicle screws 1, whereas on the left hand assembly (having a different type of fulcrum) the ligament 7 has also been applied thereto.

## Claims

1. Assembly for the stabilisation of two adjacent vertebral bodies (10) of the spine comprising of two pedicle screws (1), having a threaded shaft (2) with a tapering first end (3) for introduction into the vertebral bodies (10) and a head portion (4) with a second end (5), an elastic ligament (7) be disposed transversely to said two pedicle screws (1) and fixed to the head portions (4) of said two pedicle screws (1) at a distance y < x from the end portion of the fulcrum (8), **characterized by**
(A) a flexible longitudinal fulcrum (6) with two end portions (8), adapted to be disposed transversely to said pedicle screws (1), and fixed with its end portions (8) to the head portions (4) of said two pedicle screws (1) at a distance x from said second end.

2. Assembly according to claim 1, **characterized in that** said fulcrum (6) ist at least partially compressible in the longitudinal direction.

3. Assembly according to claim 1 or 2, **characterized in that** said distance x is in the range of 10 - 20 mm.

4. Assembly according to one of the claims 1 to 3, **characterized in that** said distance y is in the range of 7 to 17 nun.

5. Assembly according to one of the claims 1 to 4, **characterized in that** said fulcrum (6) comprises
A) a longitudinal main body (9) of which at least its middle portion consists of a flexible material ; and
B) two end portions (8) consisting of a rigid material.

6. Assembly according to claim 5, **characterized in that** said two end portions (8) are designed as a sleeve over said main body (9).

7. Assembly according to claim 5, **characterized in that** said main body (9) of said fulcrum (6) is either a high-density polyethylene (HDP) with a modulus of elasticity between 17'000 to 28'000 kg/cm² or a poly tetra-fluoro ethylene (PTFE) with modulous of elasticity between 3000 to 6500 kg/cm²

8. Assembly according to one of the claims 1 to 7, **characterized in that** said fulcrum (6) is covered with a fabric sleeve.

9. Assembly according to one of the claims 1 to 8, **characterized in that** said fulcrum (6) is drum-shaped.

10. Assembly according to one of the claims 1 to 9, **characterized in that** said head portions (4) of said pedicle screws (1) are provided with a fixation mechanism (12,14,16) for accommodating and fixing an end portion (8) of the fulcrum(6).

11. Assembly according to one of the claims 1 to 10, **characterized in that** said head portion (4) of said pedicle screw (1) is provided with a smooth seat (12) for receiving said ligament (7), a groove (14) for receiving said end portion (8) of the fulcrum (6), and a threaded portion (16) for receiving a locking nut (11).

12. Assembly according to one of the claims 1 to 11, **characterized in that** said head portion (4) is provided with a fastening mechanism (13) for tightening said end portion (8) of the fulcrum (6).

13. Assembly according to one of the claims 12, **characterized in that** said fastening mechanism (13) comprises a sleeve (17) which can be passed over the end portion (8) of the fulcrum (6), after the latter is seated into said groove (14) in the head portion (4) of said pedicle screw (1).

14. Assembly according to claim 13, **characterized in that** it comprises a locking nut (11) which can be tightened over the corresponding threaded part (16) in the head (4) of the pedicle screw (1), fastening the sleeve (17) and the head end (8) and the fulcrum to the pedicle screw(1).

15. Assembly according to one of the claims 1 to 14, **characterized in that** said ligament (7) has the shape of a closed loop, which can be passed over the second end (5) of the pedicle screw, to sit on the smooth part (12) of the head portion (4) of the pedicle screw.

16. Assembly according to one of the claims 1 to 15, **characterized in that** said ligament (7) is made up with braided polyester.

17. Assembly according to one of the claims 1 to 16, **characterized in that** it comprises a cap nut (15) which can be screwed into the second end of the pedicle screw (I) to retain the ligament (7) in place.

18. Assembly according to one of the claims 1 to 17, **characterized in that** said ligament (7 )consists of a single length flexible cord, which preferebly is made up with an outer laycr of braided polyester, and an inner core of stretchable material.

## Patentansprüche

1. Vorrichtung zur Stabilisierung zweier benachbarter Wirbelkörper (10) der Wirbelsäule, welche folgendes umfasst: zwei Pedikelschrauben (1), die jeweils einen Gewindeschaft (2) mit einem konischen, ersten Ende (3) zur Einbringung in die Wirbelkörper (10) und einen Kopfteil (4) mit einem zweiten Ende (5) aufweisen, ein elastisches Band (7), das entsprechend angepasst ist, um quer zu den beiden Pedikelschrauben (1) angeordnet zu werden und in einem Abstand y < x von dem Endteil (8) des Gelenkzapfens (6) an den Kopfteilen (4) der beiden Pedikelschrauben (1) befestigt zu werden, **gekennzeichnet durch**
einen flexiblen, longitudinal verlaufenden Gelenkzapfen (6) mit zwei Endteilen (8), die entsprechend angepasst sind, um quer zu den beiden Pedikelschrauben (1) angeordnet zu werden, wobei ersterer mit seinen Endteilen (8) an den Kopfteilen (4) der beiden Pedikelschrauben (1) in einem Abstand x von dem zweiten Ende befestigt ist.

2. Vorrichtung nach Anspruch 1, **dadurch gekennzeichnet, dass** der Gelenkzapfen (6) zumindest teilweise in Längsrichtung komprimierbar ist.

3. Vorrichtung nach Anspruch 1 oder 2, **dadurch gekennzeichnet, dass** der Abstand x in dem Bereich von 10 bis 20 mm liegt.

4. Vorrichtung nach einem der Ansprüche 1 bis 3, **dadurch gekennzeichnet, dass** der Abstand y in dem Bereich von 7 bis 17 mm liegt.

5. Vorrichtung nach einem der Ansprüche 1 bis 4, **dadurch gekennzeichnet, dass** der Gelenkzapfen (6) folgendes umfasst:
A) einen longitudinal verlaufenden Hauptkörper (9), von welchem zumindest der Mittelteil aus einem flexiblen Material besteht; und
B) zwei Endteile (8), die aus einem steifen Material bestehen.

6. Vorrichtung nach Anspruch 5, **dadurch gekennzeichnet, dass** die beiden Endteile (8) jeweils als eine sich über den Hauptkörper (9) erstreckende Hülse ausgestaltet sind.

7. Vorrichtung nach Anspruch 5, **dadurch gekennzeichnet, dass** es sich bei dem Hauptkörper (9) des Gelenkzapfens (6) entweder um ein Polyethylen hoher Dichte (HDPE) mit einem Elastizitätsmodul zwischen 17.000 und 28.000 kg/cm² oder um ein Polytetrafluorethylen (PTFE) mit einem Elastizitätsmodul zwischen 3000 und 6500 kg/cm² handelt.

8. Vorrichtung nach einem der Ansprüche 1 bis 7, **dadurch gekennzeichnet, dass** der Gelenkzapfen (6) mit einer Stoffhülle bedeckt ist.

9. Vorrichtung nach einem der Ansprüche 1 bis 8, **dadurch gekennzeichnet, dass** der Gelenkzapfen (6) trommelförmig ausgebildet ist.

10. Vorrichtung nach einem der Ansprüche 1 bis 9, **dadurch gekennzeichnet, dass** die Kopfteile (4) der Pedikelschrauben (1) jeweils mit einem Befestigungsmechanismus (12,14,16) zur Aufnahme und Fixierung eines Endteils (8) des Gelenkzapfens (6) ausgestattet sind.

11. Vorrichtung nach einem der Ansprüche 1 bis 10, **dadurch gekennzeichnet, dass** der Kopfteil (4) der Pedikelschraube (1) mit einem glatten Sitz (12) zur Aufnahme des Bandes (7), einer Rille (14) zur Aufnahme des Endteils (8) des Gelenkzapfens (6), sowie mit einem Gewindeteil (16) zur Aufnahme einer Klemmmutter (11) ausgestattet ist.

12. Vorrichtung nach einem der Ansprüche 1 bis 11, **dadurch gekennzeichnet, dass** der Kopfteil (4) mit einem Befestigungsmechanismus (13) zum Festziehen des Endteils (8) des Gelenkzapfens (6) ausgestattet ist.

13. Vorrichtung nach Anspruch 12, **dadurch gekennzeichnet, dass** der Befestigungsmechanismus (13) eine Hülse (17) umfasst, welche über den Endteil (8) des Gelenkzapfens (6) geschoben werden kann, nachdem letzterer in die in dem Kopfteil (4) der Pedikelschraube (1) ausgebildete Rille (14) eingesetzt worden ist.

14. Vorrichtung nach Anspruch 13, **dadurch gekennzeichnet, dass** sie eine Klemmmutter (11) umfasst, welche über dem entsprechenden in dem Kopf (4) der Pedikelschraube (1) ausgebildeten Gewindeteil (16) festgezogen werden kann, wodurch die Hülse 17 und der obere Endteil (8) des Gelenkzapfens an der Pedikelschraube (1) befestigt werden können.

15. Vorrichtung nach einem der Ansprüche 1 bis 14, **dadurch gekennzeichnet, dass** das Band (7) die Form einer geschlossenen Schleife aufweist, welche über das zweite Ende 5 der Pedikelschraube geschoben werden kann, so dass es auf dem glatten Teil (12) des Kopfteils (4) der Pedikelschraube (1) aufliegt.

16. Vorrichtung nach einem der Ansprüche 1 bis 15, **dadurch gekennzeichnet, dass** das Band (7) aus geflochtenem Polyester gefertigt ist.

17. Vorrichtung nach einem der Ansprüche 1 bis 16, **dadurch gekennzeichnet, dass** sie eine Hutmutter (15) umfasst, die in das zweite Ende der Pedikelschraube (1) einschraubbar ist, um das Band (7) in Position zu halten.

18. Vorrichtung nach einem der Ansprüche 1 bis 17, **dadurch gekennzeichnet, dass** das Band (7) aus einem flexiblen Strang von einfacher Länge besteht, welches vorzugsweise aus einer äusseren Schicht aus geflochtenem Polyester und einem inneren Kern aus einem dehnbaren Material gefertigt ist.

## Revendications

1. Dispositif permettant de stabiliser deux corps vertébraux voisins (10) de la colonne vertébrale, lequel comprend : deux vis pédiculaires (1) qui présentent respectivement un corps fileté (2) avec une première extrémité conique (3) destinée à être insérée dans les corps vertébraux (10) et une tête (4) avec une deuxième extrémité (5), un ligament élastique (7) qui est conçu de manière à être disposé perpendiculairement aux deux vis pédiculaires (1) et à être fixé sur les têtes (4) des deux vis pédiculaires (1) à une distance y < x de l'extrémité (8) du support articulé (6), **caractérisé par**
un support articulé flexible (6) s'étendant de manière longitudinale et comprenant deux extrémités (8) qui sont conçues de manière à être disposées perpendiculairement aux deux vis pédiculaires (1), ledit support articulé étant fixé avec ses extrémités (8) aux têtes (4) des deux vis pédiculaires (1) à une distance x de la deuxième extrémité.

2. Dispositif selon la revendication 1, **caractérisé en ce que** le support articulé (6) est compressible, au moins partiellement, dans le sens longitudinal.

3. Dispositif selon la revendication 1 ou 2, **caractérisé en ce que** la distance x est comprise entre 10 et 20 mm.

4. Dispositif selon l'une des revendications 1 à 3, **caractérisé en ce que** la distance y est comprise entre 7 et 17 mm.

5. Dispositif selon l'une des revendications 1 à 4, **caractérisé en ce que** le support articulé (6) comprend les éléments suivants :
A) un corps principal (9) s'étendant de manière longitudinale, dont au moins la partie centrale est réalisée dans un matériau flexible; et
B) deux extrémités (8) qui sont réalisées dans un matériau rigide.

6. Dispositif selon la revendication 5, **caractérisé en ce que** les deux extrémités (8) sont réalisées respectivement sous la forme d'une douille s'étendant par-dessus le corps principal (9).

7. Dispositif selon la revendication 5, **caractérisé en ce que** le corps principal (9) du support articulé (6) est soit un polyéthylène de densité élevée (HDPE) avec un module d'élasticité compris entre 17 000 et 28 000 kg/cm², soit un polytétrafluoréthylène (PTFE) avec un module d'élasticité compris entre 3 000 et 6 500 kg/cm².

8. Dispositif selon l'une des revendications 1 à 7, **caractérisé en ce que** le support articulé (6) est recouvert d'une enveloppe en tissu.

9. Dispositif selon l'une des revendications 1 à 8, **caractérisé en ce que** le support articulé (6) est réalisé sous forme de tambour.

10. Dispositif selon l'une des revendications 1 à 9, **caractérisé en ce que** les têtes (4) des vis pédiculaires (1) sont respectivement équipées d'un mécanisme de fixation (12,14,16) permettant de recevoir et de fixer une extrémité (8) du support articulé (6).

11. Dispositif selon l'une des revendications 1 à 10, **caractérisé en ce que** la tête (4) de la vis pédiculaire (1) est équipée d'un siège lisse (12) destiné à recevoir le ligament (7), d'une rainure (14) destinée à recevoir l'extrémité (8) du support articulé (6) ainsi que d'une partie filetée (16) destinée à recevoir un écrou de serrage (11).

12. Dispositif selon l'une des revendications 1 à 11, **caractérisé en ce que** la tête (4) est équipée d'un mécanisme de fixation (13) permettant de fixer par serrage l'extrémité (8) du support articulé (6).

13. Dispositif selon la revendication 12, **caractérisé en ce que** le mécanisme de fixation (13) comprend une douille (17) que l'on fait glisser par-dessus l'extrémité (8) du support articulé (6) après que ce dernier a été introduit dans la rainure (14) formée dans la tête (4) de la vis pédiculaire (1).

14. Dispositif selon la revendication 13, **caractérisé en ce qu'**il comprend un écrou de serrage (11) qui peut être fixé par serrage sur la partie filetée correspondante formée dans la tête (4) de la vis pédiculaire (1), la douille (17) et l'extrémité supérieure (8) du support articulé pouvant ainsi être fixées sur la vis pédiculaire (1).

15. Dispositif selon l'une des revendications 1 à 14, **caractérisé en ce que** le ligament (7) présente la forme d'une boucle fermée que l'on peut faire glisser par-dessus la deuxième extrémité 5 de la vis pédiculaire de telle sorte qu'elle repose sur la partie lisse (12) de la tête (4) de la vis pédiculaire (1).

16. Dispositif selon l'une des revendications 1 à 15, **caractérisé en ce que** le ligament (7) est réalisé en polyester tressé.

17. Dispositif selon l'une des revendications 1 à 16, **caractérisé en ce qu'**il comprend un écrou borgne (15) qui peut être vissé dans la deuxième extrémité de la vis pédiculaire (1) afin de maintenir le ligament (7) en position.

18. Dispositif selon l'une des revendications 1 à 17, **caractérisé en ce que** le ligament (7) est formé par une corde flexible de longueur simple qui se compose, de préférence, d'une couche extérieure en polyester tressé et d'un corps intérieur en un matériau extensible.
